(19) Europäisches Patentamt
European Patent Office
Office européen des brevets

(11) **EP 4 084 008 A1**

(12) **EUROPEAN PATENT APPLICATION**

(43) Date of publication:
**02.11.2022 Bulletin 2022/44**

(21) Application number: **21382381.8**

(22) Date of filing: **30.04.2021**

(51) International Patent Classification (IPC):
**G16H 20/40** (2018.01)  **G16H 40/63** (2018.01)
**G16H 50/50** (2018.01)  **A61B 5/08** (2006.01)
**A61M 16/00** (2006.01)

(52) Cooperative Patent Classification (CPC):
(C-Sets available)
**G16H 20/40; A61M 16/026; G16H 40/63;**
**G16H 50/50;** A61M 2016/0027; A61M 2016/0036;
A61M 2016/1025; A61M 2205/502; A61M 2230/06;
A61M 2230/202; A61M 2230/205; A61M 2230/30;
A61M 2230/432; A61M 2230/60        (Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicants:
• **Universitat Politècnica de Catalunya**
  **08034 Barcelona (ES)**
• **Universidad de Antioquia**
  **Medellín (CO)**

(72) Inventors:
• **SERNA HIGUITA, Leidy Yanet**
  **08950 Esplugues de Llobregat (ES)**
• **MAÑANAS VILLANUEVA, Miguel Ángel**
  **08902 L'Hospitalet de Llobregat (ES)**
• **HERNÁNDEZ VALDIVIESO, Alher Mauricio**
  **050010 Medellín (CO)**

(74) Representative: **Segui Quetglas, Margalida et al**
  **Torner, Juncosa i Associats, S.L.**
  **C/Pau Claris, 108, 1r 1a**
  **08009 Barcelona (ES)**

(54) **A MEDICAL VENTILATOR SYSTEM AND A METHOD FOR PREDICTING TRANSIENT STATES OF A VENTILATED PATIENT UPON CHANGES IN MECHANICAL VENTILATOR SETTINGS**

(57) A medical ventilator system and a method for predicting transient states of a ventilated patient upon changes in mechanical ventilator settings are provided. The system includes a mechanical ventilator connected to a ventilated patient; and a modeling component to receive clinical and/or physiological variables of the ventilated patient and ventilator settings. The modeling component predicts a cardiorespiratory transient state of the ventilated patient by tunning the clinical and/or physiological variables and the ventilator settings throughout the adjustment of parameters' values of several physiological submodels, generating as a result a personalized ventilated patient model. The generated personalized ventilated patient model is simulated based on new ventilator settings, a result of the simulation providing: traces of simulated output respiratory and cardiovascular signals; indexes related to features of transient and stationary phases of each simulated output physiological signal; indexes related to ventilation, respiratory rhythm, respiratory pressures, ventilatory mechanics, work of breathing, cardiac function, arterial gases.

Fig. 1

**(Cont. next page)**

(52) Cooperative Patent Classification (CPC): (Cont.)

C-Sets
A61M 2230/06, A61M 2230/005;
A61M 2230/202, A61M 2230/005;
A61M 2230/205, A61M 2230/005;
A61M 2230/30, A61M 2230/005;
A61M 2230/432, A61M 2230/005;
A61M 2230/60, A61M 2230/005

**Description**

TECHNICAL FIELD

The present invention relates to respiratory care of a patient and, more particularly, to a system and method for predicting the transient states of a ventilated patient upon changes in mechanical ventilator settings.

BACKGROUND OF THE INVENTION

The cardiorespiratory system refers to the union of the cardiovascular and respiratory systems whose elements work together to supply oxygen for cellular use and remove carbon dioxide generated by the metabolic reactions. Both subsystems involve a wide variety of mechanisms with complex and different dynamics aiming to keep the homeostasis of arterial blood gases and pH, even in pathological situations or external disturbances. The variables resulting from this complex interaction allow evaluating their correct functionality. Consequently, they have an essential role in patient diagnoses, monitoring, and treatment.

Respiratory diseases are one of the leading causes of death and disease throughout the world. A major one is Chronic Obstructive Pulmonary Disease (COPD), which is characterized by both high airway resistance and low lung distensibility. In this disease, the low lung distensibility is caused by the dynamic hyperinflation generated by the expiratory flow limitation that is, in turn, produced by the high airway resistance. This fact creates a concomitant increase of work of breathing (WOB), a functional impairment of inspiratory muscle function, and adverse effects on the patient's hemodynamics. This situation can lead to dyspnea, respiratory muscle fatigue, and, therefore, to the necessity of pharmacological or ventilatory therapies.

Respiratory failure defines the inability to maintain normal gas exchange, that is, hypercapnia and/or hypoxemia produced as a consequence of pump deficiency (i.e., impairments on the nervous system, thoracic cage, and respiratory muscles) and lung deterioration (i.e., impairments on of perfusion/diffusion and shunt), respectively. Frequently, these failures are also characterized by an increase of WOB and, therefore, the demand energy.

Patients with COPD during an acute phase of their disease require mechanical ventilation (MV) to assist or replace their pulmonary function, a procedure in which the correct ventilator setting plays an important role both in the patient's recovery and weaning.

One of the main challenges in MV is to select a proper mode and ventilator setting according to the current patient's condition, which is changing since his/her connection to the ventilator, without putting at risk the patient's life. The most critical time interval since the patient is connected or the ventilation mode or the parameters setting are changed is the following minutes and hours, from the patient's transient until stationary (steady-state) is reached. This is a laborious process due to the high quantity of available ventilatory modes, the disparity of their nomenclatures (not unified), and the patient's characteristics. In situations of high oxygen flow and high mechanical energy, MV can lead to lung-damaging, oxygen toxicity and ventilator-induced lung injury (VILI). Moreover, a weak patient-ventilator interaction causes discomfort and dyspnea, increases the need for sedative and paralytic agents, prolongs MV and intensive care unit (ICU) length of stay, and increases the likelihood of respiratory muscle injury and tracheostomy and, in some cases, the death.

Respiration and its integration with the cardiovascular system depend on optimal and integral action of several discrete anatomic units: brain, peripheral nervous system, chest wall, respiratory muscles, pleura, upper and lower airways, heart, red blood cells, and lungs. Thus, the patient's status is so complex and involves so much information that a good understanding of lung disease pathogenesis and its interaction with cardiovascular components can help clinicians to select proper settings of ventilatory therapies like mechanical ventilation. Unfortunately, only basic and standardized MV settings tend to be used in clinical practice, which misuses the benefits of advanced ones. Such settings are usually chosen based on the medical expertise of the clinicians. They are adjusted on a trial-and-error basis as the ventilated patient response is observed, increasing the iatrogenic risk. This is why smart systems and/or methods that provide clinicians goodly useful and complete information about the possible patient's cardiorespiratory under changes of both ventilation modes and parameters settings are imperative. Even experts need help in front of advanced modes in the new ventilators, so an improved training for the early and advanced stage clinicians could permit them to have a more effective intensive care service.

These tools and methods are based on mathematical and computational models. Many empirical and functional models have been published in the literature to describe numerous aspects of the cardiovascular and respiratory systems. Ventilatory stimuli like hypoxia, hypercapnia, and exercise are frequently used to evaluate the performance of such models. Despite the efforts made in the development of this kind of tools, none of them is able to adequately simulate both transient and steady-state phases of the variables involved in this system under incremental ventilatory and metabolic stimuli. The development considerations and the specificity of its application involve characteristics and restrictions that constrain the model simulation to a specific situation. For instance, the models by Fincham & Tehrani [1] and by Magosso and Ursino [2], despite involving some sophisticated regulatory mechanisms, are specialized only in the respiratory or cardiovascular system, respectively, constraining their application for a complete cardiorespiratory analysis.

On the other hand, the model by Cheng et al. [3] is one of the most comprehensive cardiorespiratory models, but its control mechanisms are mainly focused on the sleep mechanism. Similarly, the models by Albanese et al. [4] and Serna et al. [5] are also comprehensive models of the cardiorespiratory system. Although they include more detailed cardiorespiratory interactions or better neuronal control dynamics under incremental exercise, transient responses of these models but Serna et al. [5] have not strictly assessed yet. Frequently, only their steady-state responses are validated with experimental data, whereas their transient responses use to be analyzed from a qualitative approach. In particular, the study published in [5] has demonstrated that models by Fincham & Tehrani [1] and Cheng et al. [3] do not consider properly the transient state prediction when using the stimulus of exercise for their validation. Variables like arterial blood of CO2 (PaCO2) and arterial blood of O2 (PaO2) presented slow responses with transient situations representing a high risk for the simulated subject before arriving at the stationary state.

Several of these models have become patents associated with clinical decision support systems (CDSS) to help clinicians in ventilation settings. However they automatically bring the best solution, that is, the best ventilator setting without providing a complete cardiorespiratory response and taking into account only steady-state conditions but not transient period. Some of them are mentioned hereon.

The EP3238110A1 [6] discloses a medical ventilator system comprising, among others, physiological sensors configured to acquire measured values for the ventilated patient's monitored physiological variables. A fitted ventilated patient cardiopulmonary (CP) model is generated representing the ventilated patient for the monitored physiological variables. In addition, the system generates the computed values of the predictions of the fitted ventilated patient CP model for both monitored and unmonitored physiological variables. The system also comprises a ventilator optimization component to output suggested ventilator settings based on the measured values for the ventilated patient of the monitored physiological variables, the computed values for the ventilated patient of the unmonitored physiological variables, and one or more physiological parameter constraints. The suggested ventilator settings are adjusted by minimizing a cost function for the fitted ventilated patient CP model.

The input data of the system come from sensors, that is, they are real physiological signals. Thus, the approach of training is not considered but only of clinical application. The system output is the proposed ventilator settings that are based on the minimization of a cost function; thus, this is based only on the patient's steady-state and the transient one is not considered. Particularly, the patent indicates that cost function is calculated with mean values of variables over a fixed time window (e.g., one minute). Thus, the invention output is not the predicted physiological variables with the proposed ventilator settings either the patient's complete state. The patent indicates that settings output depends on the ventilator capabilities and the operating ventilation mode. It does not depend on the cardiorespiratory model which should provide much more complete information about the patient's state with the new settings, both steady and transitory states. Thus, the output information is very concise and little: only the proposed settings are important and the clinician doesn't have complete forecasting information to evaluate the convenience of changing the settings to the proposed ones. Even the invention considers the possibility to bypass the clinician to change the ventilator settings automatically (called "closed-loop approach"). This is why the invention is considered to be integrated into the ventilator or connected via cable or wireless with the ventilator.

The EP3191987B1 [7] relates to a mechanical ventilation system for respiration of an associated patient with decision support for lung ventilator settings. Particularly, the system allows assessing the ventilator settings effects, especially for positive end expiratory pressure (PEEP), on model parameters associated with lung mechanics and gas exchange of a ventilated patient. It includes, among others, a control means adapted for using both data from the patient (measured) and data from physiological models, able to provide decision support in relation to a suggested PEEP value. Thus, this invention is considered only for clinical application with real patients and not for training clinicians.

In addition, the optimal ventilator settings can be found from model simulation of clinical outcome variables according to, for example, a set of clinical preference functions. Thus, the resulting PEEP of the system only considers steady-state patient response and does not include transitory aspects that allows predicting the patient's spontaneous breathing. The system output is the effect on one or more model parameters for changes in the ventilator settings, particularly, for PEEP. Thus, no information about cardiorespiratory variables is provided to the operator or clinician in steady or transient states.

The EP3068286A1 [8] discloses a decision support system for lung ventilator settings. In particular, a mechanical ventilation system for respiration aid of an associated patient. This comprises, among others, measurement means of parameters of inspired and expired gas and blood values of said associated patient. The output of the system is a graphical user interface (GUI) where at least one pair of scoring values which have conflicting effects is arranged for being displayed: a first scoring value related to over-ventilation and a second one related to under-ventilation of the associated patient. Like the EP3191987B1, this system also includes preference functions describing clinical preference (barotrauma, hypoxia, acidosis-alcalosis, and oxygen toxicity) which are used to calculate the score values. The model included in the system comprises a set of equations for respiratory control and gas exchange formulated under steady-state conditions; therefore, the system does not consider the patient's response assessment from a dynamic standpoint, that is, transient state. The invention is based on several measurement means; thus, this is considered only for clinical decision support system not for training. This invention does not enable the clinical to get an overview of the cardiorespiratory variables of a patient in relation to current or future ventilator settings. Indeed, inventors consider it too complex to manage so much information and that is why outcomes are only a pair of score values. Inventors state that these scoring values simplify the decision phase for the clinician for when and how action should be taken.

The EP2029209B1 [9] discloses a system and a method for monitoring the ventilation support provided by a ventilator. That is, supplying breathing gas to a patient via a breathing circuit that is in fluid communication with the lungs of the patient. The method comprises among others: (1) a monitoring system with at least one sensor adapted to monitor the patient and each sensor generates an output signal based on the measured ventilation support parameter, (2) operator interface for operator input signals which comprise at least one of the group consisting of patient identification information; patient diagnostic information; type and size of patient airway access; anthropometric data, and (3) a processing subsystem adapted to receive the at least one of the output signals and/or at least one operator input signal, to determine suggested ventilation settings; the intelligence subsystem can comprise a trainable neural network or rule-based modules.

The desired level settings of the ventilator setting controls may be displayed on the visual display for use by the physician. The stored ventilation data output signal, and particularly the subset of the ventilation data output signal containing the ventilation data, which is used by the intelligent subsystem in the determination of the desired level setting of the controls, may be provided to the visual display. Thus, system outcomes do not include the cardiorespiratory signals resulting from applying the suggested ventilator settings. Although the system further comprises evaluating time history of the patient measured signals, it does not generate computed time-domain signals for the ventilated patient with the new ventilator settings. The invention is based on an intelligent system trained by a clinician according to the best solution and his/her criteria or rules determined by him/her.

In any case, the predicting cardiorespiratory signals are estimated. Again, the invention includes at least one sensor connected to the patient, thus this is designed to be applied in clinical practice but not for training the clinician.

In summary, in MV, transient-states can be interpreted as the path that the patient carries out before arriving at the stationarity state, i.e., before adapting to a new ventilator setting. Intubated patients are frequently patients in a critical situation who are unconscious or who cannot breathe on their own, so they don't have the ability to interfere with the ventilatory therapy to change their current state, affecting their comfort and possibility of having a fast recovery. At this point, it is necessary not only to guarantee good results in the long-term but also guarantee that the patient's transition to that new final state is safe and comfortable. Therefore, accounting with systems or methods that allow predicting their transient response, especially upon ventilator setting changes, will assist clinicians to properly manage the MV and also to learn how to do it in the case of early stage clinicians with a poor experience in intensive care. In this regard, many hypothetical or theoretical situations, which in clinical practice would be difficult to achieve, would become real events or phenomena that would help the healthcare staff to address the problems or the current patient state in an analytical, leisurely, methodical and condition-free manner, reducing the possibility of producing iatrogenesis during their professional practice.

In conclusion, it is well known that the assisted ventilation is a complex issue and the selection of the appropriate ventilator settings and how to change them in a specific patient is a difficult task. All patents in the field of CDSS for mechanical ventilation try to simplify this action providing directly to the clinician these settings based on the automatic minimization of cost functions or clinical preference functions. In the case of EP3068286A1 [8], for example, the only output is a pair of scores associated with over and under-ventilation. The patent EP3191987B1 [7] shows only the effects for ventilator settings, especially for PEEP, on model parameters associated with lung mechanics and gas exchange of a ventilated patient.

It is clear that all patents are focused on helping the clinician by reducing the information or giving directly the solution. The present invention has an opposite approach: it trusts on the knowledge and skills of the clinician but he/she needs complete information about what happens if ventilator settings are changed. This information includes how cardiorespiratory variables change since the settings are modified, that is, the transient response. This is a forecasting method which will permit the clinician to decide the convenience of changing the ventilator settings without a trial and error procedure in the real patient. Whole information can be provided in a GUI to select and navigate among the different cardiorespiratory variables according to the necessity of the clinician and depending on the associated patient. The clinician will be able to predict different scenarios or ventilator settings before taking a decision.

All patents mentioned above but EP2029209B1 [8] calculate their output considering steady-state conditions and that patent does not calculate the patient's response upon ventilator settings change because the system is trained previously by physicians according to their knowledge and experience, that is, once the system has learned, it is able to propose settings to other cases or clinicians.

Finally, all patents consider at least one sensor to measure a cardiorespiratory variable from a patient. Thus, they are considered to be used in clinical practice on real scenarios of intensive or semicritical units among others. The approach of using the system to train clinicians in simulated scenarios with input cardiorespiratory signals from a database, for example, is not considered.

DESCRIPTION OF THE INVENTION

The present invention aims to provide a clinical forecasting method that allows predicting the patient's transient state of cardiorespiratory variables upon changes in their ventilatory therapy. This method is a solution for the problem exposed hereon, which is related to an integral evaluation of patient's adaptation to new ventilator settings, i.e., the assessment of not only of final patient response but also of the patient adaptation process (transient state).

Embodiments of the present invention provide according to a first aspect a medical ventilator system for predicting transient states of a ventilated patient upon changes in mechanical ventilator settings. The system comprises a mechanical ventilator connected to a ventilated patient to provide ventilation to the ventilated patient according to ventilator settings; and a modeling component comprising one or more processors and at least one memory and configured to receive information of clinical and/or physiological variables of the ventilated patient, and the ventilator settings. The modeling component is configured to predict the cardiorespiratory transient and steady-state response of the ventilated patient by tuning one or more parameters' values of a plurality of physiological mathematical submodels for an initial ventilator settings; said one or more parameters' values being coefficients of differential equations (it should be noted that the decision to modify one or another value is determined by the sensitivity of the parameter to small variations), and the plurality of physiological submodels comprising lung mechanics, gas exchange in the lungs of the ventilated patient, circulatory system, respiratory control and/or cardiovascular control, generating as a result of said tuning a personalized ventilated patient model; and simulating the generated personalized ventilated patient model based on one or more new ventilator settings requested by an operator, the new ventilator settings being simulated until their stabilization, a result of the simulation providing traces of simulated output respiratory and cardiovascular signals; indexes related to features of transient and stationary phases of each simulated output physiological signal; and/or indexes related to ventilation, respiratory rhythm, respiratory pressures, ventilatory mechanics, work of breathing, cardiac function and/or arterial gases assessment.

In an embodiment, the proposed system further comprises sensors; the clinical and/or physiological variables of the ventilated patient being acquired by these sensors. Alternatively or complementary, the system also comprises a database; thus the physiological variables of the ventilated patient being stored in the database before being transmitted to the modeling component.

In an embodiment, the system also has a graphical user interface, the result of the simulation being provided through the graphical user interface.

The clinical and/or physiological variables can comprise anthropometric data of the ventilated patient, a current clinical state of the ventilated patient and/or monitored values of physiological signals of the ventilated patient, among others. The ventilator setting and/or the new ventilator settings can comprise a minute ventilation, a respiratory rate, a tidal volume, an inspiratory time, an inspiratory airflow flow, a control pressure ventilation, a support pressure ventilation, an inspiratory trigger sensitivity, an expiratory trigger sensitivity, a positive end-expiratory pressure, a continuous positive airway pressure and a inspiratory fraction of $O_2$, among others.

Other embodiments of the invention that are disclosed herein also include software programs to perform a method to predict transient states of a ventilated patient upon changes in mechanical ventilator settings. More particularly, a computer program product is one embodiment that has a computer-readable medium including computer program instructions encoded thereon that when executed on at least one processor in a computer system causes the processor to perform the operations indicated herein as embodiments of the invention.

In an embodiment, the computer program can include instructions that score a success of the predicted cardiorespiratory transient state and to report said success. Moreover, the score can be stored in a memory or database, or even shared among different microprocessors remotely connected or with a cloud computing system.

In an embodiment, the one or more new ventilator settings further include scores based on clinical targets related to ventilation, oxygenation and work of breathing. Thus, the one or more ventilator settings can be used, in a patient's future state, to evaluate (following the clinical target medical criteria previously established) which one is better and show to clinician by a visual warning the possibility to change the ventilator setting in order to improve the patient state.

In another embodiment, the personalized ventilated patient model is continuously updated as new clinical and/or physiological variables and new ventilator settings arrive or are inputted. In this case, the clinical and/or physiological variables can be compared with the result of the simulation. A warning signal can be generated if a result of the comparison establishes that the result of the simulation is better in terms of lower work of breathing or respiratory effort, avoiding asynchronies or other similar.

The invention can be used for different purposes. For example, as a method for training early-stage clinicians in basic and advanced contents related to mechanical ventilation management under different respiratory patient's situations. In this case, input data of patient signals and current ventilator settings are from a database and appropriate for simulated learning exercises. Another approach would be as a method for clinical practice at intensive or semi-critical care units when predicting the personalized cardiorespiratory response of an associated patient upon mechanical ventilator setting changes. In this case, input data of patient signals and current ventilator settings are real data from medical devices connected to the patient such as vital signals monitor or mechanical ventilator, that is, in a real scenario. Other approaches also include using the method for training or educational purposes; therefore, students or early-stage clinicians can practice with the invention by simulation and gain experience to then be able to use the medical ventilator system on real patients.

BRIEF DESCRIPTION OF THE DRAWINGS

The previous and other advantages and features will be more fully understood from the following detailed description of embodiments, with reference to the attached figures, which must be considered in an illustrative and non-limiting manner, in which:

Fig. 1 schematically illustrates an embodiment of the architecture of the present invention.

Fig. 2 illustrates another embodiment of the invention's architecture.

Fig. 3 illustrates a model schematic block diagram. The dashed lines are inputs to the model. Blocks with underlined names correspond to the mechanisms added or are related to modifications made by the present invention.

DETAILED DESCRIPTION OF PREFERRED EMBODIMENTS

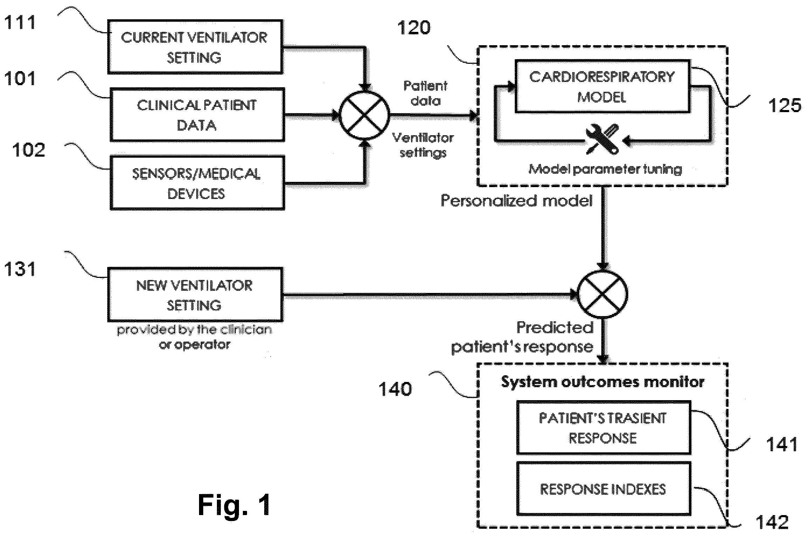

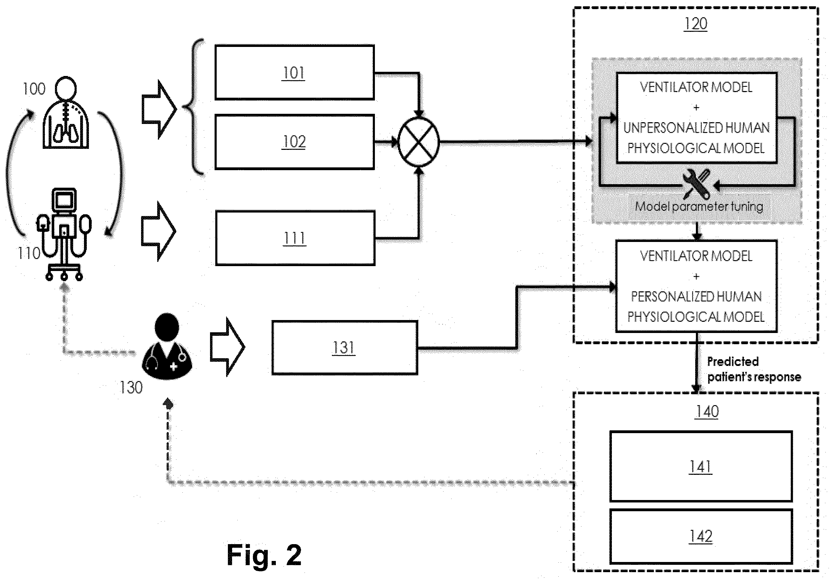

Figs. 1 and 2 graphically illustrate embodiments of the invention's architecture for predicting transient states of a ventilated patient upon changes in mechanical ventilator settings. The invention can consider input data associated with a ventilated patient 100 and their current ventilator settings 111 and can predict the patient's complete response to future ventilator settings 131. For that, it particularly includes a (dynamic) modeling component (DCRM) 120 configured or programmed to predict both those variables related to the cardiovascular and exchange gases systems and those related to the breathing control, among others. This last feature allows reproducing the patient's spontaneous ventilation (i.e., patient's breathing pattern). There, model parameters are tuned to adjust the model to the current patient situation in order to get a personalized model component 125 that can be used to simulate and predict the patient's response to other ventilator settings (also provided as input data by a clinician/operator 130). Finally, the outcome is data associated with the patient's transient response under the new ventilator settings 131: traces of cardiorespiratory variables since the settings are changed 141, indexes calculated from these traces and from the patient's state 142 and, optionally, at least one score about utility or success of the forecasting.

The DCRM inputs can comprise information related to: the current ventilator setting 111 such as the ventilatory mode and pressure or volume controlled through airflow supply to the patient 100 via circuit ventilator either directly measured or provided by a ventilator 110 or user; the patient's clinical data such as anthropometric data, clinical history, current clinical state or diagnosis, among others; the patient's monitored physiological signals that are provided by either a microprocessor/computer, connected to ventilator 110 via USB or wireless connection, or physiological sensors connected to patient 100; and the new ventilator settings 131 to be test for patient's transient state prediction upon MV changes such as control volume, control pressure, minute ventilation, respiratory frequency, inspiratory/expiratory relationship etc., introduced by the clinician/operator 130.

The sensors may include a flowmeter to measure the patient's proximal flow and pressure, an oxygen sensor to monitor the oxygen concentration provided to the patient 100, and a capnographer to non-invasively measure the patient carbon dioxide. Sensors may further include sensors related to patient cardiac variables like arterial blood pressure, heart rate, oxygen saturation, among others. In other embodiments, system inputs can come from a connectivity platform capable of acquiring clinical data from any medical device monitoring or supporting a critical or semi-critical patient.

The DCRM component 120 is based on a self-regulated cardiorespiratory model that comprises several physiological features, such as cardiovascular circulation, respiratory mechanics, gas exchange system as well as cardiovascular and respiratory controllers. In general, the DRCM component 120 can include some or all of these physiological features and can optionally be adapted to include other kinds of features.

In DRCM 120, every subsystem and controller is based on previous physiological models and incorporates reported models of several mechanisms related to the aerobic exercise dynamics. The proper transient response has been obtained thanks to relationships build from ordinary differential equations, changes in several of their coefficients, that is, model parameters, and adding features and subsystems/blocks to the model presented in [5], in particular:

- The estimation of ventilatory demand as a function of brain carbon dioxide partial pressure ($PbCO_2$), $PaO_2$, $PaCO_2$,

and metabolically related neural drive component to the ventilation (MRV).

- A respiratory control approach focused on mechanical work of breathing (WOB) minimization.

- The action of the central command on cardiovascular efferent sympathetic and parasympathetic control activities.

- The influence of the central respiratory neuromuscular drive on cardiovascular efferent sympathetic and parasympathetic control activities.

- A separate description of vascular beds in active and resting skeletal muscles.

- The local vasodilatation of the active muscles.

- The effect of muscular contractions on venous return (Muscle Pump).

- The effect of respiration on venous return (Respiratory Pump).

The DCRM component 120 particularly includes a respiratory controller that is based on the adaptation of the model by Fincham and Tehrani [1] made in the study by Serna et al. [5], and by a cardiovascular controller that includes mechanisms related to response to patient metabolic activity. These mechanisms comprise the effect of respiration on sympathetic and parasympathetic activity according to proposed by Cheng et al. [3] and the action mechanism of central command proposed by Magosso and Ursino [2]. The effect of respiration through the central respiratory neuromuscular drive, arising from the respiratory centers and modulated by the respiratory auto-rhythmicity and ventilatory demand, influences the response in the efferent pathways through a directly proportional effect on sympathetic activity and inversely proportional on parasympathetic activity. The central command mechanism evaluated as the metabolic regulation response, determined as a function of the aerobic threshold (AT) and production values both at rest and exercise, has a direct additive effect on the effector pathways, exciting the sympathetic activity and inhibiting the parasympathetic activity, and an indirect vasodilator effect in response to the absence of oxygen over the systemic peripheral vessels related to the active skeletal muscles.

Additionally, the DCRM component 120 further includes a ventilator model from which ventilator pressure resulting from the ventilator setting information provided to the system can be simulated. The ventilator model includes conventional ventilatory strategies like, but not limited, assisted/mandatory and intermittent modes controlled by either pressure or volume as well as a spontaneous mode supported by pressure. It may further include more sophisticated modes like pressure augmentation (PA) and pressure-regulated volume control (PRVC) modes, or advanced ventilatory strategies like the adaptive support ventilation (ASV), the neural adjust ventilation assist (NAVA), and the proportional adaptive ventilation (PAV), among others. The ventilator model can be parametrized from ventilator setting information. In this case, the ventilator setting comprises but not limited to: minute ventilation (VE), respiratory rate (RR), tidal volume (VT), inspiratory time (TI), inspiratory airflow flow (Vi), control pressure ventilation (PCV), support pressure ventilation (PSV), inspiratory trigger sensitivity (ITS), expiratory trigger sensitivity (ETS), positive end-expiratory pressure (PEEP), continuous positive airway pressure (CPAP) and inspiratory fraction of $O_2$. Optionally, the ventilator model can further include features related to the circuit to the patient in order to implement capabilities related to tube compensation in intubated patients.

The DCRM component 120 comprises linear and nonlinear mathematical equations (in some embodiment more than two hundred) including differential equations. Such equations allow computing transient responses of both monitoring and no-monitoring physiological variables (i.e. variables that cannot be directly measured by the sensors) and interrelationships among them. Therefore, patient status is evaluated following a dynamic approach, which considers not only those variables that are directly monitored, but also those ones implicated in the underlying processes of the cardiac and respiratory systems. Moreover, it has the advantage of predicting the ventilated patient response to changes in the ventilator settings 111, so patient-ventilator interaction can be also evaluated.

DCRM component 120 can further implement a fitting, or tunning, algorithm that allows, from the current ventilator settings 111, the personalization of the cardiorespiratory model to a specific patient throughout the estimation of the parameters' values that minimize the differences between the model predictions and the monitored physiological variables (such as heart rate, airway pressure, muscle pressure, arterial partial pressures of $O_2$ and $CO_2$, blood pressure, etc.). A suitable approach to minimize these differences comprises global or local optimization techniques. A specific optimization algorithm is, but not limited to, the evolutionary strategy with covariance matrix and adaptation (CMA-ES), a stochastic global optimization algorithm based on adaptive and evolutionary strategies having had good results of convergence speed, precision, and accuracy when has been used in mathematical models of human physiological systems.

The fitting algorithm involves a high complexity due to a large number of the model parameters that need to be identified. Thus, the system involves a parameter selection procedure that allows previously identifying the most suitable parameters to be adjusted. Such a procedure aims to reduce the complexity of the fitting process by identifying a reduced set of parameters whose modification is justified according to the experimental or monitored physiological variables. This procedure particularly includes two stages comprising:

1. Complexity reduction selecting parameters according to the role in the model and the conditions of fitting and validation. This approach is based on the parameters' classification into five roles that are generally found in physiological system models, corresponding to: (i) time constants, parameters related to temporal characteristics of the model mechanisms, and usually associated with delays or characteristics of the transfer functions; (ii) conversion parameters, which are constant values related to equivalences between units of measure; (iii) covariates, corresponding to values that allow to define simulation conditions regarding to disturbances, characteristics of the environment and the population to be simulated; (iv) Initial values, corresponding to initial estimates of the model variables, usually required as integration limits and whose action mainly affects the temporal characteristics of the responses; and (v) gain and thresholds, related to the response in magnitude of the model's mechanisms. Particularly, only time constants, initial values and gain and threshold parameters are considered for the following stages

2. Sensitivity analysis aiming to identify, from the parameters left at this stage, the most sensitive parameters. For this, two approaches are used.

- The first one based on the relative sensitivity, i.e. the sensitivity of each physiological variable regarding each model parameter. In this approach, most appropriate parameters to be adjusted will be either those that have higher effects on the variable or those whose effect is mainly in only one variable. This approach allows making easier the later adjustment forward obtaining the personalized DCRM 120.

- The second one is based on the total sensitivity of the model regarding each parameter, that is, the global effect in all physiological variables under slight changes in the model preselected parameters.

Tables 1 and 2 list and define some, but not limited, of the parameters to be adjusted after this sensitivity analysis, i.e. the parameters with the highest relative and total sensitivity respectively. In Table 1, variables related to each parameter are also shown.

Table 1: List of some of the most relative sensitive parameters identified in the first stage of the parameter selection procedure (see text)

| Variable | Parameter | Definition |
|---|---|---|
| HR | HPbasal | Basal value for HP for denervated heart |
| | Vtot | Total amount of blood volume contained in the circulatory system |
| | Gps | Parasympathetic Gain varied with sleep drive |
| PaCO2 | Vdead_0 | Offset value of dead space volume |
| | VArest | Basal value for alveolar ventilation |
| | GVdead | Constant gain for dead space volume |
| PaO2 | KcMRV | Constant gain of central response to exercise (neural drive) |
| | a2 | Parameter in CO2 dissociation equation |
| | MRBCO2 | Metabolic production rate for CO2 in the brain tissue |
| PS | Pn | Center pressure for sigmoidal function |
| | KErv | Parameter of end-diastolic pressure-volume function of right ventricule |
| | Rsa | Systemic arterial flow resistance |

(continued)

| Variable | Parameter | Definition |
|---|---|---|
| PD | PaCO2_n | Nominal arterial CO2 partial pressure i |
| | C1 | Maximum concentration of hemoglobin-bound oxygen (to be adjusted) |
| | alpha2 | Parameter in CO2 dissociation equation |
| VE | A_0_ua | Maximum area of opening in upper airway (sensitivity) |
| | K_ua | Proportionality coefficient between Aua and Yua |
| | beta2 | Parameter in CO2 dissociation equation |
| BF | lambdal | Breathing pattern controller parameter |
| | a1 | Parameter in O2 dissociation equation |
| | Pcrit_awake | Critical upper airway pressure in wakefulness |

Table 2: List of some of the most global sensitive parameters identified in the second stage of the parameter selection procedure

| Parameter | Definition |
|---|---|
| Kbg | Ventilation controller, constant blood-gas disociation |
| KcCO2 | Constant gain of CO2 central chemoreceptors |
| C2 | Maximum carbon dioxide concentration (to be adjusted) |
| KpCO2 | Constant gain of CO2 peripheral chemoreceptors |
| Pmax | Breathing pattern controller parameter |

Once these parameters have been found, any parameter adjustment of the DCRM component 120 to obtain the personalized model component 125 is carried out under two sequential estimation stages. The first stage, corresponding to a general fitting, is based on a traditional approach in which the model is fitted by the parameters obtained from the total sensitivity analysis (listed above in Table 1). As a result, a significant decrease in the total prediction error of the model is obtained, but the errors respect to a specific variable can be still high and be reduced. The second stage corresponds to a fine fitting of the model and is based on the adjustment by the parameters listed above in Table 2 in the relative analysis for decreasing the prediction error in those variables of interest.

Once the model parameters are estimated, response of the patient 100 to changes in mechanical ventilator setting 111 can be predicted. For this, new ventilator settings 131 are provided by physician/operator 130.

At the systems outcome monitor 140, traces of cardiorespiratory variables 141 since the settings are changed and indexes 142 calculated from these traces and from the patient's state are visualized in a monitor display. Indexes 142 can include features related to transient (constant time, peak value, peak time, overshoot, etc.) and stationary phases (final value) of each monitored physiological variable. Indexes 142 can further comprise those ones related to ventilation, respiratory rhythm, respiratory pressures, ventilatory mechanics, work of breathing, cardiac function and arterial gases.

In an embodiment, the proposed system further includes a set of stored instructions comprising: reading acquired physiological variables of the ventilated patient 100; fitting techniques for tuning the DCRM parameters and generating a personalized ventilated patient DCRM 125, to reproduce by simulation the current patient state with the current ventilator settings 111; and predicting patient's complete response for new ventilator setting 131 using the personalized ventilated patient DCRM 125.

In an embodiment, the system further comprises a storage component to store a portion of the actual and simulated patient's data, the current 111 and new 131 ventilator settings, and at least one score associated with the utility of the system outcomes and/or the success of the forecasting for being later applied to the patient 100 in reality. This score will be requested from the system or method to the clinician 130, who will manually enter at least one score at the end of the execution. This score or scores can be further presented to another clinician as a system outcome when the patient's data and the actual and proposed ventilator settings are similar to those scores or scores that were stored in order to provide feedback from other colleagues. Similarities among scores can be evaluated by a microprocessor using techniques such as, but not limited to, supervised algorithms or multidimensional correlation of the indexes considered in the forecasting method. If several past predictions are considered similar to the current one, statistics of such scores will be presented to the clinician, for example, in average. Finally, stored data can be shared by different microprocessors that are remotely connected or available within a cloud computing environment.

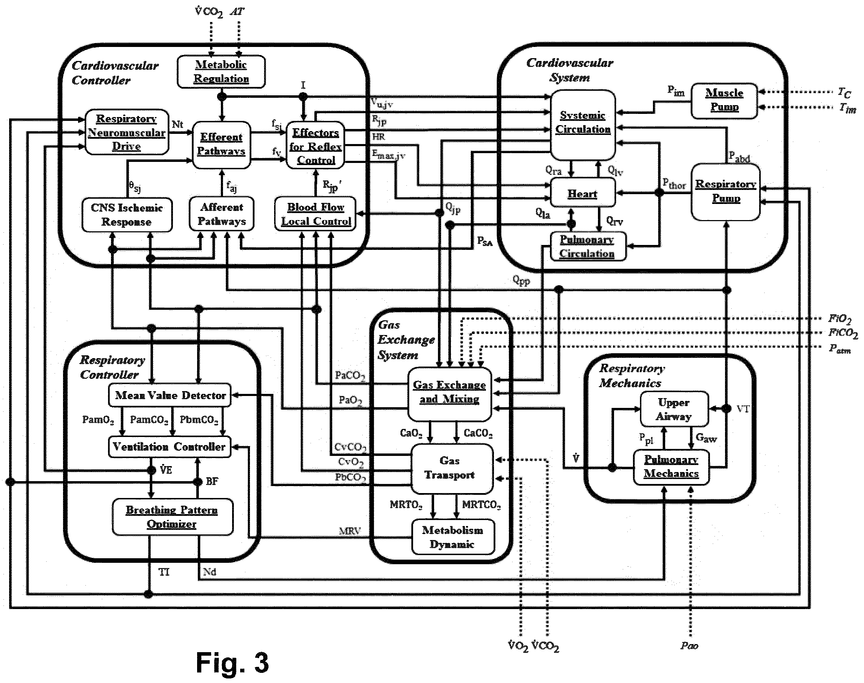

With reference now to Fig. 3, therein different models executed by the DCRM component 120 are illustrated, according to an embodiment. The following table 3 indicates the symbols description.

Table 3: Symbols' description of the block diagram of Symbols' description of DCRM

| Symbol | Description |
|---|---|
| $CaCO_2$ | Carbon dioxide concentration in the arterial blood |
| $CaO_2$ | Oxygen concentration in the arterial blood |
| $CvCO_2$ | Carbon dioxide concentration in the venous blood |
| $CvO_2$ | Oxygen concentration in the venous blood |
| $E_{max,jv}$ | Regulated end-systolic elastance of each ventricle |
| $f_{aj}$ | Afferent activity from arterial baroreceptors, chemoreceptors, and lung-stretch receptors |
| $FiCO_2$ | Inspired fractions of dry carbon dioxide |
| $FiO_2$ | Inspired fractions of dry oxygen |
| $f_{sj}$ | Activity in efferent sympathetic fibers directed to arterioles, veins, and heart |
| $f_v$ | Efferent vagal activity |
| $G_{aw}$ | Airway flow gain factor |
| $I$ | The action of central command |
| $MRTCO_2$ | Tissues carbon dioxide metabolic ratio |
| $MRTO_2$ | Tissues oxygen metabolic ratio |
| $MRV$ | Metabolically related neural drive component to the ventilation |
| $Nd$ | Neural drive signal |
| $Nt$ | Central respiratory neuromuscular drive response |
| $P_{abd}$ | Abdominal pressure |
| $PamCO_2$ | Mean value of $PaCO_2$ in each respiratory cycle |
| $PamO_2$ | Mean value of $PaO_2$ in each respiratory cycle |
| $Poo$ | The pressure at the airway opening |
| $PbCO_2$ | Brain carbon dioxide partial pressure |
| $PbmCO_2$ | Mean value of $PbCO_2$ in each respiratory cycle |
| $P_{im}$ | Intramuscular pressure |
| $P_{pl}$ | Intrapleural pressure |
| $P_{sa}$ | Systemic arterial pressure signal |
| $P_{thor}$ | Intrathoracic pressure |
| $Q_{jp}$ | Cardiac output of each peripheral compartment |
| $Q_{la}$ | Cardiac input to the left atrium |
| $Q_{lv}$ | Cardiac output from left ventricle |
| $Q_{pp}$ | Cardiac output from the pulmonary peripheral compartment |

(continued)

| Symbol | Description |
|---|---|
| $Q_{ra}$ | Cardiac input to the right atrium |
| $Q_{rv}$ | Cardiac output from left ventricle |
| $R_{jp}$ | Regulated resistance of each peripheral compartment |
| $R'_{jp}$ | Regulated resistance by the blood flow local control for each peripheral compartment |
| $T_C$ | Overall duration of the muscular contraction |
| $T_{im}$ | Time duration of the muscular contraction-relaxation cycle |
| $V_{u,jv}$ | Regulated unstressed volume of each venous compartment |
| $\theta_{sj}$ | Offset term representing the effect of the CNS ischemic response on the sympathetic fibers directed to peripheral circulation, veins, and heart |

Changes performed in the equations of DCRM regarding the other submodels published in the literature:

1- Cardiovascular system/submodel

The cardiovascular system submodel is based on Ursino and Magosso's [10] model and modifications from the same authors [2]. The added mechanisms include:

• a separate description of vascular beds of active and resting skeletal muscles

• a functional description of local metabolic vasodilation into active muscles and its effect on the venous return, and

• the effect of respiration on the venous return of compartments located inside the thoracic and abdominal cavities.

The active skeletal muscle compartment's addition involves dividing the peripheral and venous circulation into six compartments arranged in parallel. It involves the inclusion of equations that relate their pressure and flow during rest and their control mechanisms.

This model does not implement the description of the thoracic cavity proposed by Magosso and Ursino [2]. Instead, the vena cava flow and pressure descriptions implemented by Cheng et al. [3] and based on the work proposed by Lu et al. [3] were included.

2- Cardiovascular controller/submodel

The cardiovascular controller implemented is also primarily based on Ursino and Magosso's publications [10], [11].

Mechanisms related to response to aerobic exercise were included in this controller:

• The effect of respiration on sympathetic and parasympathetic activity, according to proposed by Cheng et al. [3] and
• the action mechanism of central command proposed by Magosso and Ursino [2].

3- Respiratory mechanics/submodel

The respiratory mechanics model is mostly based on the adaptation of the model by Cheng et al. [3] made in the study by Serna et al. [5]. It comprises two compartments representing the pulmonary and upper airway mechanics. The modifications were implemented in the pulmonary mechanics, specifically in the muscle pressure wave and the air movement equation. The implemented muscle pressure equation is the one proposed by Poon et al. [12], which is modeled as a quadratic function during inspiration and an exponential function during expiration. The parameter values that define these waveforms are respiratory controller regulation results and are received in this system as an input named neural drive response (Nd). The volume and airflow signals are related to the muscle pressure signal according to the air movement equation proposed by M. Younes and W. Riddle [13]-[15].

4- Respiratory controller/submodel

The respiratory controller is based on the adaptation of Fincham and Tehrani's model (16) presented in the study by Serna et al. [5]. Information provided by the central chemoreceptors (PbCO2), peripheral chemoreceptors (PaO2 and PaCO2), and the metabolically related neural drive component to the ventilation (MRV) is used to estimate the ventilatory demand of the subject (i.e., the needed ventilation to keep arterial gases between their basal values).

Instead of Otis' optimization approach [16], from which BF is calculated based on a steady-state minimization of work of breathing (WOB) principle in [5], DCRM incorporates the optimization approach formulated in (39) wherein breathing pattern is adjusted each cycle for a specific level of ventilation (set-point) by minimizing cycle-cycle WOB. It includes dynamic elements to relate the neural activity with ventilatory mechanics (i.e., neuro-mechanics efficiency factors) [13]-[15], and it distinguishes between the respiratory mechanical work carried out during inspiration (WI) and expiration (WE). So, it does fit not only the ventilation but also those variables associated with the overall breathing pattern, VT, BF, and inspiratory (TI) and expiratory (TE) times.

5- Gas exchange system/submodel

The present model's gas exchange system is broadly based on the work proposed by Serna et al. [5]. It consists of an adaptation of the work of Cheng et al. [3] that includes a separate description of the oxygen consumption and CO2 production in both brain and tissues, a detailed description of the gas exchange at the cerebral level, and the determination of the dead space volume as a function of the alveolar flow. The cardiovascular system and respiratory mechanics interact through this system to regulate the concentration of blood gases.

Modifications of the cardiac output were made to obtain more realistic results of the transport and exchange of blood gases in the brain and tissues. Instead of assigning a total cardiac output percentage to the brain and tissue perfusion (0.85QT and 0.15 QT, respectively), the tissues and the brain's specific cardiac output signals were taken directly from the cardiovascular system.

Changes performed in the coefficient values of the DCRM concerning the already published submodels:

This section describes how the parameter values of the DCRM for each included system were assigned to adapt the different integrated systems/submodels, controllers, and mechanisms. The parameter values were mostly taken from previous publications (from each model's systems and controllers), except for some modifications associated with some mechanisms included in the model, like exercise.

Some parameter modifications have been assigned following the patient's anthropometric features, where the values modifications aim to achieve the equivalence. Only the most significant changes are presented below.

1- Cardiovascular system parameters

The cardiovascular system's parameter values, corresponding to resistances, compliances, and unstressed volumes, have been taken mainly from the model reported by Cheng et al. [3]. However, some of them were modified due to the addition of the active skeletal muscle compartments. The distinction between passive and active muscle compartments was applied by Magosso and Ursino [2] and aimed to evaluate the effect of the muscles' local vascular control processes during exercise on the cardiovascular response. The modifications were made following the conservative strategy proposed by Magosso and Ursino [2], where, according to the proportion of each parameter for each compartment, the total resistances, compliances, and unstressed volumes values provide the same ones reported previously by Cheng et al. [3] and Serna et al. [5]. The modified parameter values regarding the work by Cheng et al. [3] can be found in Table 4.

Table 4: Modified parameters related to the cardiovascular system

| Unstressed volume, ml | Hydraulic resistan mmHg · s/ml | Compliance, ml/mmHg |
|---|---|---|
| $V_{u,amp}$ = 63.48 | $R_{amp,0}$ = 3.51 | $C_{amp}$ = 0.315 |
| $V_{u,rmp}$ = 42.3 | $R_{rmp,0}$ = 5.27 | $C_{rmp}$ = 0.21 |
| $V_{u,amv0}$ = 301.96 | $R_{amv,n}$ = 0.0833 | $C_{amv}$ = 9.4 |
| $V_{u,rmv0}$ = 201.30 | $R_{rmv,n}$ = 0.125 | $C_{rmv}$ = 6.28 |
| $V_{u,sv0}$ = 1435.4 | $R_{sp,0}$ = 2.49 | |
| $V_{u.ev0}$ = 640.73 | $R_{ep,0}$ = 1.655 | |

$V_{u,amp}$ and $V_{u,rmp}$ are the peripheral unstressed volume in active and resting skeletal muscle; $V_{u,amv0}$, $V_{u,rmv0}$, $V_{u,sv0}$ and $V_{u,ev0}$ are the basal level of venous unstressed volume in active and resting skeletal muscle, splanchnic and extra-splanchnic circulation, respectively; $R_{amp,0}$, $R_{rmp,0}$, $R_{sp,0}$ and $R_{ep,0}$ are the peripheral resistance in active and resting skeletal muscle, splanchnic and remaining extra-splanchnic vascular beds, respectively; $R_{amv,n}$ and $R_{rmv,n}$ are the venous resistance in active and resting skeletal muscle, respectively; $C_{amp}$ and $C_{rmp}$ are the peripheral compliance in active and resting skeletal muscle, respectively; $C_{amv}$ and $C_{rmv}$ are the venous compliance in active and resting skeletal muscle, respectively.

2- Cardiovascular controller parameters

The cardiovascular control system's parameter assignment depends on every included mechanism: central nervous system ischemic response, afferent pathways, and efferent neural pathways. Regarding values used in the DCRM component, we highlight:

- Parameter values mostly come from the work by Cheng et al. [3].
- Some are related to the central command mechanism derived from previous works by Magosso and Ursino ([2], [10], [11]).
- Parameter values from Albanese et al. [4] that showed the better performance to predict heart rate and systemic arterial pressure were considered.

Table 5 shows the values of parameters taken from the work of Albanese et al. [4].

Table 5: Modified parameters of the cardiovascular controller

| Central Nervous System (CNS) Ischemic Response | | |
|---|---|---|
| $k_{isc,sh}$ = 6 $mmHg$ | $x_{sh}$ = 53 $spikes\,/s$ | |
| **Afferent Pathway** | | |
| $G_{ap}$ = 11.76 $spikes \cdot l^{-1} \cdot s^{-1}$ | | |
| **Efferent Pathways** | | |
| $W_{c,sp}$ = 1.716 | $W_{p,sp}$ = -0.3997 | $W_{b,sp}$ = -1.1375 |
| $W_{c,sv}$ = 1.716 | $W_{p,sv}$ = -0.3997 | $W_{b,sv}$ = -1.1375 |
| $W_{c,sh}$ = 1 | $W_{p,Sh}$ = 0 | $W_{b,sh}$ = -1.75 |
| $W_{c,v}$ = 0.2 | $W_{p,v}$ = -0.103 | |

$k_{isc,sh}$ is a gain parameter related to the heart sympathetic activity; $x_{sh}$ is a saturation for the offset of heart sympathetic activity; $G_{ap}$ is a constant gain related with the lung stretch receptors reflex; $W_{c,sp}$, $W_{c,sv}$, $W_{c,sh}$ and $W_{c,v}$ are the chemoreflex gain for the peripheral resistance, unstressed venous volume, heart sympathetic and parasympathetic activity, respectively; $W_{p,sp}$, $W_{p,sv}$, $W_{p,sh}$ and $W_{p,v}$ are the lung stretch receptor reflex gain for the peripheral resistance, unstressed venous volume, heart sympathetic and parasympathetic activity, respectively; $W_{b,sp}$, $W_{b,sv}$ and $W_{b,sh}$ are the carotid baroreceptor gain for the peripheral resistance, unstressed venous volume and heart sympathetic activity, respectively.

3- Respiratory mechanics parameters

The parameter values of the respiratory mechanics mostly were taken from Serna et al. [5], using for the overall resistance ($R_{rs}$) and elastance ($E_{rs}$) the healthy people values reported by Poon et al. [12]. According to this change, the compartmental values ($R_{CW}$, $R_L$, $R_{AW}$, $E_{CW}$, $E_L$) were calculated by maintaining a proper proportion. The modified values are shown in Table 6.

Table 6: Modified parameters of respiratory mechanics

| Resistance, $cmH_2O \cdot s/l$ | Elastance, $cmH_2O/l$ |
|---|---|
| $R_{rs}$ = 3.02 | $E_{rs}$ = 21.9 |
| $R_{CW}$ = 0.8326 | $F_{cw}$ = 10.545 |

(continued)

| Resistance, $cmH_2O \cdot s/l$ | Elastance, $cmH_2O/l$ |
|---|---|
| $R_L$ = 1.3661 | $E_L$ = 10.545 |
| $R_{AW}$ = 0.82128 | |

$R_{CW}$ is the chest wall resistance; $R_L$ is the lung transmural resistance; $R_{AW}$ is the airway wall resistance; $E_{CW}$ is the Chest wall elastance; $E_L$ is the lung transmural elastance.

4- Respiratory controller parameters

The parameter values of the respiratory controller correspond to those published by Serna et al. [17]. Specifically, those related to the inspiratory and expiratory WOB weighting factors, the respiratory efficiency factors, and their non-linear variation (see Table 5). The efficiency factors allow considering the effects of respiratory-mechanical limitation and decrease neuro-mechanics efficiency with increasing respiratory efforts [12]. They depend on the parameter $P_{max}$ and $\dot{P}_{max}$, whose values are those reported for a healthy subject by Serna et al. in [17] and Poon et al. in [12], respectively. Table 7 shows the used parameter values.

Table 7: Parameters of the respiratory controller

| Breathing Pattern Optimizer | | |
|---|---|---|
| $\lambda_1$ = 0.860 | $\lambda_2$ = 0.489 | $n$ = 1.101 |
| $P_{max}$ = 50 $cmH_2O$ | $\dot{P}_{max}$ = 1000 $cmH_zO$ /s | |

$\lambda_1$ and $\lambda_2$ are weighing factors of the inspiratory and expiratory work; $n$ defines the non-linear variation of the mechanical plant efficiency; $P_{max}$ is the maximum inspiratory muscle pressure; $\dot{P}_{max}$ is the maximum pressure rate during inspiration.

5- Gas exchange system parameters

The gas exchange system's parameter values correspond to those used by Serna et al. [5]. Most of them are based on the work of Cheng et al. [3] and Fincham and Tehrani [1], but others were modified considering average values of the experimental data (see the reference work for more detail). Table 8 shows the values of the modified parameters in the work of Serna et al. [5].

Table 8: Modified parameters of the gas exchange system

| Gas Exchange and Mixing | | |
|---|---|---|
| $\beta_1$ = 0.008275 | | |
| **Gas Transport in the brain compartment** | | |
| $MRBCO_2$ = 0.0009 $l$ /s STPD | $KCSFCO_2$ = 320 $s$ | $h$ = 0.0183 $ml/(100g \cdot s)$ |
| $MRBO_2$ = 0.000925 $l$ /s STPD | $KCCO_2$ = 346000 $s \cdot cm^{-2} \cdot l^{-1}$ | $dc$ = 0.015 $cm$ |
| **Gas transport in body tissues compartment** | | |
| $VTCO_2$ = 15 $l$ | $MRTCO_2 basal$ = 0.0033333 $l$ /s STPD | $MRCO_2$ = 0.016667 $l$ /s STPD |
| $VTO_2$ = 6 $l$ | $MRTO_2 basal$ = 0.0041667 $l$ /s STPD | $MRO_2$ = 0.020833 $l$ /s STPD |
| $\tau MR$ = 50 $s$ | | |

$\beta_1$ is a parameter associated with the Bohr and Haldane effects regarding the $O_2$ dissociation in blood; $MRBCO_2$ and $MRBO_2$ are the metabolic production rate for $CO_2$ and $O_2$ in the brain tissue, respectively; $KCCO_2$ is the $CO_2$ central receptor constant; $KCSFCO_2$ is the $CO_2$ diffusion time constant of cerebrospinal fluid; $h$ is the cerebral blood flow constant; $d$ is the depth of central receptor below the surface of the medulla; $VTCO_2$ and $VTO_2$ are the body tissue storage volume for $CO_2$ and $O_2$, respectively; $MRTCO_2 basal$ and $MRTO_2 basal$ are the basal metabolic production rate for $CO_2$ and consumption rate for $O_2$, respectively; $MRCO_2$ and $MRO_2$ are the rates of production and consumption of $CO_2$ and $O_2$, respectively; $\tau MR$ is the metabolic rate time constant. STPD is standard temperature and pressure, dry.

Various aspects of the method for predicting transient states of a ventilated patient upon changes in mechanical ventilator settings, as described herein, may be embodied in programming. Program aspects of the technology may be thought of as "products" or "articles of manufacture" typically in the form of executable code and/or associated data that is carried on or embodied in a type of machine readable medium. Tangible non-transitory "storage" type media include any or all of the memory or other storage for the computers, processors, or the like, or associated modules thereof, such as various semiconductor memories, tape drives, disk drives and the like, which may provide storage at any time for the software programming.

All or portions of the software may at times be communicated through a network such as the Internet or various other telecommunication networks. Such communications, for example, may enable loading of the software from one computer or processor into another, for example, from a management server or host computer of a scheduling system into the hardware platform(s) of a computing environment or other system implementing a computing environment or similar functionalities in connection with image processing. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to tangible "storage" media, terms such as computer or machine "readable medium" refer to any medium that participates in providing instructions to a processor for execution.

A machine-readable medium may take many forms, including but not limited to, a tangible storage medium, a carrier wave medium or physical transmission medium. Nonvolatile storage media include, for example, optical or magnetic disks, such as any of the storage devices in any computer(s), or the like, which may be used to implement the system or any of its components shown in the drawings. Volatile storage media may include dynamic memory, such as a main memory of such a computer platform. Tangible transmission media may include coaxial cables; copper wire and fiber optics, including the wires that form a bus within a computer system. Carrier-wave transmission media may take the form of electric or electromagnetic signals, or acoustic or light waves such as those generated during radio frequency (RF) and infrared (IR) data communications. Common forms of computer-readable media may include, for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a physical processor for execution.

Those skilled in the art will recognize that the present teachings are amenable to a variety of modifications and/or enhancements. For example, although the implementation of various components described herein may be embodied in a hardware device, it may also be implemented as a software only solution-e.g., an installation on an existing server. In addition, image processing as disclosed herein may be implemented as a firmware, firmware/software combination, firmware/hardware combination, or a hardware/firmware/software combination.

The present disclosure and/or some other examples have been described in the above. According to descriptions above, various alterations may be achieved. The topic of the present disclosure may be achieved in various forms and embodiments, and the present disclosure may be further used in a variety of application programs. All applications, modifications and alterations required to be protected in the claims may be within the protection scope of the present disclosure.

The scope of the present invention is defined in the following set of claims.

**References:**

[1] W. F. Fincham and F. T. Tehrani, "A mathematical model of the human respiratory system," J. Biomed. Eng., vol. 5, no. 2, pp. 125-133, Apr. 1983, doi: 10.1016/0141-5425(83)90030-4.

[2] E. Magosso and M. Ursino, "Cardiovascular response to dynamic aerobic exercise: A methematical model," Med. Biol. Eng. Comput., vol. 40, no. 6, pp. 660-674, Nov. 2002, doi: 10.1007/BF02345305.

[3] L. Cheng, O. Ivanova, H. H. Fan, and M. C. K. Khoo, "An integrative model of respiratory and cardiovascular control in sleep-disordered breathing," Respir. Physiol. Neurobiol., vol. 174, no. 1-2, pp. 4-28, 2010, doi: 10.1016/j.resp.2010.06.001.

[4] A. Albanese, L. Cheng, M. Ursino, and N. W. Chbat, "An integrated mathematical model of the human cardiopulmonary system: Model development," Am. J. Physiol. - Hear. Circ. Physiol., vol. 310, no. 7, pp. H899-H921, 2016, doi: 10.1152/ajpheart.00230.2014.

[5] L. Y. Serna, M. A. Mañanas, A. M. Hernández, and R. A. Rabinovich, "An Improved Dynamic Model for the Respiratory Response to Exercise," Front. Physiol., vol. 9, Feb. 2018, doi: 10.3389/fphys.2018.00069.

[6] A. Albanese, N. W. Chbat, R. Buizza, and N. Karamolegkos, "Systems and methods for model-based optimization of mechanical ventilation." Google Patents, 2018.

[7] D. S. Karbing, S. E. Rees, and J. B. Jensen, "Mechanical ventilation system for respiration with decision support." Google Patents, 2017.

[8] S. E. Rees and D. S. Karbing, "Decision support system for lung ventilator settings." Google Patents, 2016.

[9] M. J. Banner, R. E. I. I. Neil, J. C. Principe, and P. B. Blanch, "Ventilator monitor system and method of using same." Google Patents, 2007.

[10] M. Ursino and E. Magosso, "Acute cardiovascular response to isocapnic hypoxia. I. A mathematical model," Am. J. Physiol. Circ. Physiol., vol. 279, no. 1, pp. H149-H165, Jul. 2000, doi: 10.1152/ajpheart.2000.279.1.H149.

[11] E. Magosso and M. Ursino, "A mathematical model of CO 2 effect on cardiovascular regulation," Am. J. Physiol. Circ. Physiol., vol. 281, no. 5, pp. H2036-H2052, Nov. 2001, doi: 10.1152/ajpheart.2001.281.5.H2036.

[12] C. S. Poon, S. L. Lin, and O. B. Knudson, "Optimization character of inspiratory neural drive," J. Appl. Physiol., vol. 72, no. 5, pp. 2005-2017, May 1992, doi: 10.1152/jappl.1992.72.5.2005.

[13] M. Younes and W. Riddle, "A model for the relation between respiratory neural and mechanical outputs. I. Theory," J. Appl. Physiol., vol. 51, no. 4, pp. 963-978, Oct. 1981, doi: 10.1152/jappl.1981.51.4.963.

[14] M. Younes, W. Riddle, and J. Polacheck, "A model for the relation between respiratory neural and mechanical outputs. III. Validation," J. Appl. Physiol., vol. 51, no. 4, pp. 990-1001, Oct. 1981, doi: 10.1152/jappl.1981.51.4.990.

[15] W. Riddle and M. Younes, "A model for the relation between respiratory neural and mechanical outputs. II. Methods," J. Appl. Physiol., vol. 51, no. 4, pp. 979-989, Oct. 1981, doi: 10.1152/jappl.1981.51.4.979.

[16] A. B. Otis, W. O. Fenn, and H. Rahn, "Mechanics of Breathing in Man," J. Appl. Physiol., vol. 2, no. 11, pp. 592-607, May 1950, doi: 10.1152/jappl.1950.2.11.592.

[17] L. Y. Serna Higuita, M. A. Mananas, A. Mauricio Hernandez, J. Marina Sanchez, and S. Benito, "Novel Modeling of Work of Breathing for Its Optimization During Increased Respiratory Efforts," IEEE Syst. J., vol. 10, no. 3, pp. 1003-1013, Sep. 2016, doi: 10.1109/JSYST.2014.2323114.

**Claims**

**1.** A medical ventilator system for predicting transient states of a ventilated patient upon changes in mechanical ventilator settings, comprising:

a mechanical ventilator (110) connected to a ventilated patient (100) to provide ventilation to the ventilated patient (100) according to ventilator settings (111);
a modeling component (120) comprising one or more processors and at least one memory and configured to receive clinical and/or physiological variables (101, 102) of the ventilated patient (100), and the ventilator settings (111),

**characterized in that:**
the modeling component (120) is configured to predict a cardiorespiratory transient state of the ventilated patient (100) by:

tuning the clinical and/or physiological variables (101, 102) get from the ventilated patient (100) and the ventilator settings (111) throughout the adjustment of one or more parameters' values of a plurality of physiological submodels, said one or more parameters' values being coefficients of differential equations related to transient-states and/or steady-states, and the plurality of physiological submodels comprising lung mechanics, gas exchange in the lungs of the ventilated patient (100), circulatory system, respiratory control and/or cardiovascular control, generating as a result of said tuning a personalized ventilated patient model (125); and

simulating the generated personalized ventilated patient model (125) based on one or more new ventilator settings (131) requested by an operator (130), the new ventilator settings (131) being simulated until their stabilization, a result of the simulation providing:

- traces of simulated output respiratory and cardiovascular signals;
- indexes related to features of transient and stationary phases of each simulated output physiological signal;
- indexes related to ventilation, respiratory rhythm, respiratory pressures, ventilatory mechanics, work of breathing, cardiac function and/or arterial gases, obtained from the simulated output physiological signals.

2. The system of claim 1, further comprising sensors, wherein the clinical and/or physiological variables (101, 102) get from the ventilated patient (100) being acquired by the sensors.

3. The system of claim 1, further comprising a database, wherein the clinical and/or physiological variables (101, 102) get from the ventilated patient (100) being stored in the database before being transmitted to the modeling component (120).

4. The system of claim 1, further comprising a graphical user interface, the result of the simulation being provided through the graphical user interface.

5. The system of claim 1, wherein the clinical and/or physiological variables (101, 102) comprise anthropometric data of the ventilated patient (100), a current clinical state of the ventilated patient (100) including arterial blood gases and/or treatment drugs and/or monitored values of physiological signals of the ventilated patient (100).

6. The system of claim 1, wherein the ventilator setting (111) and/or the new ventilator settings (131) include at least one of: a minute ventilation, a respiratory rate, a tidal volume, an inspiratory time, an inspiratory airflow flow, a control pressure ventilation, a support pressure ventilation, an inspiratory trigger sensitivity, an expiratory trigger sensitivity, a positive end-expiratory pressure, a continuous positive airway pressure and an inspiratory fraction of $O_2$.

7. A non-transitory storage medium storing instructions readable and executable by one or more processors to perform a method to predict transient states of a ventilated patient upon changes in mechanical ventilator settings, the method comprising:

receiving clinical and/or physiological variables (101, 102) of a ventilated patient (100) and ventilator settings (111) of a mechanical ventilator (110) to which the ventilated patient (100) is connected;
predicting a cardiorespiratory transient state of the ventilated patient (100) by:

tuning the clinical and/or physiological variables (101, 102) get from the ventilated patient (100) and the ventilator settings (111) throughout the adjustment of one or more parameters' values of a plurality of physiological submodels, said one or more parameters' values being coefficients of differential equations related to transient-states and/or steady-states, and the plurality of physiological submodels comprising lung mechanics, gas exchange in the lungs of the ventilated patient (100), circulatory system, respiratory control and/or cardiovascular control, generating as a result of said tuning a personalized ventilated patient model (125); and
simulating the generated personalized ventilated patient model (125) based on one or more new ventilator settings (131) requested by an operator (130), the new ventilator settings (131) being simulated until their stabilization, a result of the simulation providing:

- traces of simulated output respiratory and cardiovascular signals;
- indexes related to features of transient and stationary phases of each simulated output physiological signal;
- indexes related to ventilation, respiratory rhythm, respiratory pressures, ventilatory mechanics, work of breathing, cardiac function and/or arterial gases, obtained from the simulated output physiological signals.

8. The non-transitory storage medium of claim 7, wherein the instructions are further configured to score a success of the predicted cardiorespiratory transient state and to report said success.

9. The non-transitory storage medium of claim 8, wherein the instructions are further configured to store said score in a memory or database.

10. The non-transitory storage medium of claim 8 or 9, wherein the instructions are further configured to share the score with different microprocessors remotely connected or by a cloud computing system.

11. The non-transitory storage medium of any of the previous claims 7 to 10, wherein the instructions are further configured to continuously update the personalized ventilated patient model (125) as the clinical and/or physiological variables (101, 102) and the new ventilator settings (131) change.

12. The non-transitory storage medium of claim 11, wherein the instructions are further configured to compare the clinical and/or physiological variables (101, 102) with the result of the simulation, and to generate a warning signal when a result of the comparison establishes that the result of the simulation is better at least in terms of lower work of breathing or respiratory effort or avoiding asynchronies.

13. The non-transitory storage medium of claim 11, wherein the instructions are further configured to compare the clinical and/or physiological variables (101, 102) with the result of the simulation, and to generate a warning signal when the variables show a worse patient's situation and a result of the comparison establishes new ventilator settings to the result of the simulation is maintaining the better previous patient's condition at least in terms of lower work of breathing or respiratory effort or avoiding asynchronies.

Fig. 1

**Fig. 2**

Fig. 3

Europäisches
Patentamt

European
Patent Office

Office européen
des brevets

# EUROPEAN SEARCH REPORT

Application Number

EP 21 38 2381

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MAGOSSO E. ET AL: "A theoretical study of the transient and steady-state cardiorespiratory response to exercise", ENVIRONMENTAL HEALTH RISK V, vol. 1, 22 August 2005 (2005-08-22), pages 17-26, XP055851268, Southampton, UK ISSN: 1747-4485, DOI: 10.2495/BIO050021 ISBN: 978-1-84564-201-3 * the whole document, in particular paragraphs [0001], [0008]-[0038], [0046], [0055]-[0064], [0073], [0077], and figures 1-12 * | 1-13 | INV. G16H20/40 G16H40/63 G16H50/50 A61B5/08 A61M16/00 |
| X | Antonio Albanese: "Physiology-based Mathematical Models for the Intensive Care Unit: Application to Mechanical Ventilation", , 31 December 2014 (2014-12-31), XP055332137, ISBN: 978-1-303-94555-7 Retrieved from the Internet: URL:https://academiccommons.columbia.edu/d ownload/fedora_content/download/ac:199653/ CONTENT/Albanese_columbia_0054D_12039.pdf [retrieved on 2017-01-04] * page 1 - page 10 * * page 31 - page 100 * * page 117 - page 155 * * page 167 - page 203 * | 1-13 | TECHNICAL FIELDS SEARCHED (IPC) G16H A61M A61B |
| X | EP 3 238 110 B1 (KONINKLIJKE PHILIPS NV [NL]) 7 April 2021 (2021-04-07) * page 2, paragraph 0001 - page 6, paragraph 0037 * | 1-13 | |

The present search report has been drawn up for all claims

| Place of search | Date of completion of the search | Examiner |
|---|---|---|
| Munich | 14 October 2021 | Sasa Bastinos, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 38 2381

This annex lists the patent family members relating to the patent documents cited in the above-mentioned European search report.
The members are as contained in the European Patent Office EDP file on
The European Patent Office is in no way liable for these particulars which are merely given for the purpose of information.

14-10-2021

| Patent document cited in search report | | Publication date | Patent family member(s) | Publication date |
|---|---|---|---|---|
| EP 3238110 | B1 | 07-04-2021 | BR 112017013436 A2 | 06-03-2018 |
| | | | CN 107209797 A | 26-09-2017 |
| | | | EP 3238110 A1 | 01-11-2017 |
| | | | JP 6730990 B2 | 29-07-2020 |
| | | | JP 2017538553 A | 28-12-2017 |
| | | | RU 2017126240 A | 24-01-2019 |
| | | | US 2018001042 A1 | 04-01-2018 |
| | | | WO 2016103142 A1 | 30-06-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3238110 A1
- EP 3191987 B1
- EP 3068286 A1
- EP 2029209 B1

**Non-patent literature cited in the description**

- **W. F. FINCHAM ; F. T. TEHRANI.** A mathematical model of the human respiratory system. *J. Biomed. Eng.,* April 1983, vol. 5 (2), 125-133
- **E. MAGOSSO ; M. URSINO.** Cardiovascular response to dynamic aerobic exercise: A methmatical model. *Med. Biol. Eng. Comput.,* November 2002, vol. 40 (6), 660-674
- **L. CHENG ; O. IVANOVA ; H. H. FAN ; M. C. K. KHOO.** An integrative model of respiratory and cardiovascular control in sleep-disordered breathing. *Respir. Physiol. Neurobiol,* 28 April 2010, vol. 174 (1-2
- **A. ALBANESE ; L. CHENG ; M. URSINO ; N. W. CHBAT.** An integrated mathematical model of the human cardiopulmonary system: Model development. *Am. J. Physiol. - Hear. Circ. Physiol.,* 2016, vol. 310 (7), H899-H921
- **L. Y. SERNA ; M. A. MAÑANAS ; A. M. HERNÁNDEZ ; R. A. RABINOVICH.** An Improved Dynamic Model for the Respiratory Response to Exercise. *Front. Physiol.,* February 2018, vol. 9
- **A. ALBANESE ; N. W. CHBAT ; R. BUIZZA ; N. KARAMOLEGKOS.** Systems and methods for model-based optimization of mechanical ventilation. *Google Patents,* 2018
- **D. S. KARBING ; S. E. REES ; J. B. JENSEN.** Mechanical ventilation system for respiration with decision support. *Google Patents,* 2017
- **S. E. REES ; D. S. KARBING.** Decision support system for lung ventilator settings. *Google Patents,* 2016
- **M. J. BANNER ; R. E. I. I. NEIL ; J. C. PRINCIPE ; P. B. BLANCH.** Ventilator monitor system and method of using same. *Google Patents,* 2007
- **M. URSINO ; E. MAGOSSO.** Acute cardiovascular response to isocapnic hypoxia. I. A mathematical model. *Am. J. Physiol. Circ. Physiol.,* July 2000, vol. 279 (1), H149-H165
- **E. MAGOSSO ; M. URSINO.** A mathematical model of CO 2 effect on cardiovascular regulation. *Am. J. Physiol. Circ. Physiol.,* November 2001, vol. 281 (5), H2036-H2052
- **C. S. POON ; S. L. LIN ; O. B. KNUDSON.** Optimization character of inspiratory neural drive. *J. Appl. Physiol.,* May 1992, vol. 72 (5), 2005-2017
- **M. YOUNES ; W. RIDDLE.** A model for the relation between respiratory neural and mechanical outputs. I. Theory. *J. Appl. Physiol.,* October 1981, vol. 51 (4), 963-978
- **M. YOUNES ; W. RIDDLE ; J. POLACHECK.** A model for the relation between respiratory neural and mechanical outputs. III. Validation. *J. Appl. Physiol.,* October 1981, vol. 51 (4), 990-1001
- **W. RIDDLE ; M. YOUNES.** A model for the relation between respiratory neural and mechanical outputs. II. Methods. *J. Appl. Physiol.,* October 1981, vol. 51 (4), 979-989
- **A. B. OTIS ; W. O. FENN ; H. RAHN.** Mechanics of Breathing in Man. *J. Appl. Physiol.,* May 1950, vol. 2 (11), 592-607
- **L. Y. SERNA HIGUITA ; M. A. MANANAS ; A. MAURICIO HERNANDEZ ; J. MARINA SANCHEZ ; S. BENITO.** Novel Modeling of Work of Breathing for Its Optimization During Increased Respiratory Efforts. *IEEE Syst. J.,* September 2016, vol. 10 (3), 1003-1013